# EUROPÄISCHE PATENTSCHRIFT

(11) **EP 1 010 622 B1**
(45) Veröffentlichungstag und Bekanntmachung des Hinweises auf die Patenterteilung: **17.09.2003**
(21) Anmeldenummer: 99124163.9
(22) Anmeldetag: 03.12.1999
(51) Int. Cl.: B65B 51/00

(54) **Verfahren zum Verschliessen des einen Endes einer zylindrischen Verpackungshülse, insbesondere einer Tamponverpackung, sowie Vorrichtung hierfür**
Method and device for closing one end of a cylindrical packaging tube, especially a package for a tampon
Procédé et dispositif pour fermer une extrémité d'un manchon cylindrique d'emballage, spécialement un emballage pour un tampon

(30) Priorität: 16.12.1998 DE 19858116
(43) Veröffentlichungstag der Anmeldung: 21.06.2000
(73) Patentinhaber: Ruggli Projects AG, 6332 Hagendorn Cham (CH)
(72) Erfinder: Kuhn, Kurt, 8175 Windlach (CH)
(74) Vertreter: Christophersen, Ulrich Rudolf

(56) Entgegenhaltungen:
- EP-A- 0 254 541
- DE-A- 1 955 600

## Beschreibung

Die Erfindung betrifft zunächst ein Verfahren zum Verschließen des einen Endes einer zylindrischen Verpackungshülse, insbesondere einer Verpackungshülse zur Aufnahme eines Tampons für die Frauenhygiene, durch Durchmesserreduzierung der Verpackungshülse nahe deren Ende bis zur Bildung einer geschlossenen Kappe, und Ansiegeln des außerhalb der Kappe verbliebenen Endabschnittes der Verpackungshülse an die Außenseite der Kappe.

Des weiteren betrifft die Erfindung eine Vorrichtung zum Verschließen des einen Endes einer zylindrischen Verpackungshülse, insbesondere einer Verpackungshülse zur Aufnahme eines Tampons für die Frauenhygiene, mit Mitteln zur Durchmesserreduzierung der Verpackungshülse nahe deren freien Endes bis zur Bildung einer geschlossenen Kappe.

Viele Massenartikel müssen bis zu ihrem bestimmungsgemäßen Gebrauch zum Schutz vor Verschmutzung und anderen schädlichen Einflüssen verpackt werden. Zu diesen Artikeln gehören auch Tampons für die Frauenhygiene, die jeweils einzeln in einer zylindrischen Verpackungshülse untergebracht werden. Die Bedeutung der Verpackung bei Tampons beruht auch auf dem Umstand, daß diese durch allseitiges Zusammenpressen eines feuchtigkeitsabsorbierenden Materials hergestellt werden und deshalb bei längerer Lagerung dazu neigen, infolge der Luftfeuchtigkeit wieder in allen Richtungen zu expandieren. Deshalb ist es zwecksmäßig, die Tampons jeweils mit einem reißfesten Verpackungsmaterial, z. B. Folie auf Zellulose-Basis, zu umgeben,

Aus der DE 1 955 600 C sind ein Verfahren und eine Vorrichtung zum Verschließen des einen Endes einer Verpackungshülse für Tampons bekannt. Hierzu wird das noch offene Ende einer zylindrischen Verpackungshülse, in der sich ein Tampon befindet, wobei dessen abgerundetes Einführende dem offenen Ende der Verpackungshülse zugewandt ist, durch Verzwirbeln des Endes verschlossen. Dies geschieht, indem eine mit einem zentralen Dorn sowie in Richtung auf diesen Dorn verfahrbaren Greifbacken versehene Zwirbelvorrichtung das Verpackungsmaterial am offenen Ende der Hülse erfaßt und bei feststehender Verpackungshülse dreht. Hierdurch entsteht an diesem Ende der Verpackungshülse ein Zwirbel. Anschließend lösen sich die Greifbacken, und der zentrale Dorn einschließlich der Greifbacken wird zurückgefahren. Danach ist der Zwirbel gezwungen, seine durch das Verdrehen eingenommene Form soweit als möglich zu wahren und sich möglichst nicht wieder aufzuzwirbeln. Denn erst in einem weiteren Schritt, nachdem die Verpackungshülse in eine andere Bearbeitungsposition verfahren wurde, wird die von dem Zwirbel abstehende Rosette durch einen Stempel an die Verpackungshülse angedrückt und hierbei thermisch angebügelt. Erst hierdurch wird der Zwirbel fixiert. Das so verschlossene Ende der Verpackungshülse entspricht dann im wesentlichen der Form des abgerundeten Einführendes des Tampons.

Die zur Durchführung dieses Verfahrens verwendete Vorrichtung arbeitet mit einem hohen apparatetechnischen Aufwand, da zum Erreichen des Zwirbelns eine gesteuerte Drehbewegung erforderlich ist, und ferner gesteuerte Greifbacken betätigt werden müssen, um das freie Ende der Verpackungshülse zu ergreifen und während des Zwirbelns festzuhalten.

Der Erfindung liegt die **Aufgabe** zugrunde, das Verfahren zum Verschließen des einen Endes einer zylindrischen Verpackungshülse so weiterzuentwickeln, daß sich dieses mit einer konstruktiv einfacher aufgebauten Vorrichtung durchführen läßt. Es soll ferner eine geeignete Vorrichtung geschaffen werden.

Zur **Lösung** wird hinsichtlich des Verfahrens nach Anspruch 1 vorgeschlagen, daß das Material der Verpackungshülse zur Erzielung der Durchmesserreduzierung durch eine zu der Verpackungshülse hin gerichtete Bewegung gerafft und in zusammengerafftem Zustand erhitzt wird, wobei die Erhitzung durch Beheizung über die Raffbacken erfolgt.

Das Zusammenraffen erfolgt durch eine zu der Verpackungshülse hin gerichtete Bewegung der Raffbacken, wobei die Beheizung über die Raffbacken erfolgt. Der Vorteil des Raffens des Materials der Verpackungshülse mittels Raffbacken besteht in dem konstruktiv einfach zu realisierenden Bewegungsablauf zur Erzielung der gewünschten Durchmesserreduzierung der Verpackungshülse an deren Ende. Demgegenüber erfordert ein Verzwirbeln des Endes, wie es aus dem Stand der Technik bekannt ist, einen höheren gerätetechnischen Aufwand.

Eine bevorzugte Weiterbildung des erfindungsgemäßen Verfahrens ist gekennzeichnet durch eine radial auf die Längsmittelachse der Verpackungshülse ausgerichtete Bewegung der Raffbacken. Eine solche Bewegung läßt sich konstruktiv mit einfachen Mitteln erzielen, insbesondere dann, wenn zwei einander gegenüberliegende und zwischen sich die Verpackungshülse einschließende Raffbacken vorhanden sind, welche simultan aufeinander zu bzw. voneinander weg bewegbar sind. Eine solche Bewegung kann z. B. durch eine beiden Raffbacken gemeinsame Führung realisiert werden.

Mit einer weiteren Ausgestaltung des Verfahrens wird vorgeschlagen, daß für die Zeitdauer des Zusammentreffens ein in Bezug auf die Verpackungshülse axial verfahrbarer Dorn in das offene Ende der Verpackungshülse hineinragt. Der Dorn bewirkt, daß der nach dem Raffen entstehende offene Endabschnitt der Verpackungshülse die Form einer aufgespreizten Rosette annimmt, die sich dann in einem nachfolgenden Verfahrensschritt an die Außenseite der gebildeten Kappe der Verpackungshülse ansiegeln läßt.

Die Raffbacken können permanent beheizt sein und fahren vorzugsweise so schnell zusammen, daß ein vorzeitiges Verkleben nicht stattfindet.

Bei Durchführung des Verfahrens kann sich in der Verpackungshülse bereits der zu verpackende Tampon befinden. In einer Ausgestaltung des Verfahrens wird demgegenüber jedoch vorgeschlagen, daß sich bei Durchführung des Verfahrens in der Verpackungshülse nicht der Tampon, sondern ein Formkörper befindet, der im Bereich der Kappe die Gestalt des zu verpackenden Tampons aufweist. Dieser Formkörper kann z. B. fester Bestandteil der Vorrichtung sein. in diesem Fall wird also zunächst die Verpackungshülse erfindungsgemäß so vorgeformt, wie es zur Aufnahme des Einführendes des Tampons erforderlich ist, und erst in einer sich anschließenden Verpackungsstufe wird der Tampon in die so vorbereitete Verpackungshülse eingeführt, bevor schließlich auch das andere Ende der Verpackungshülse verschlossen wird.

Des weiteren wird in Bezug auf die Durchführung des Verfahrens vorgeschlagen, daß der außerhalb der Kappe verbliebene Endabschnitt der Verpackungshülse an die Außenseite der Kappe angesiegelt wird, vorzugsweise durch kombinierte Beaufschlagung mit Hitze und Druck.

Für die Qualität der Verpackung wichtig ist auch, daß sich der Ort der Kappenherstellung stets auf derselben Länge der Verpackungshülse befindet. Um dies zu erreichen, wird mit einer Ausgestaltung des Verfahrens vorgeschlagen, daß während des Zusammenraffens nur der die Verpackung bildende Teil der Verpackungshülse in axialer Richtung festgehalten wird, wohingegen dem offenen Endabschnitt der Verpackungshülse axiale Bewegungen möglich sind.

Zur Lösung der Aufgabe der Erfindung wird femer hinsichtlich einer Vorrichtung nach Anspruch 8 vorgeschlagen, daß die Mittel zur Durchmesserreduzierung quer zur Längsachse der Verpackungshülse geführte und zu der Verpackungshülse hin bewegliche Raffbacken sind, und daß die Raffbacken beheizbar sind.

Um den axialen Abstand zwischen Kappe und Endabschnitt gering zu halten und damit das spätere Ansiegeln des Endabschnittes an die Kappe zu vereinfachen, können die Raffbacken mit einander zugewandten Gleitflächen versehen sein, wobei sich die Raffbacken über die Gleitflächen unmittelbar gegeneinander abstützen. Auf diese Weise lassen sich die Raffbacken besonders flach, d. h. mit geringer axialer Ausdehnung gestalten.

Um auch bei Verwendung von nur zwei Raffbacken eine gleichmäßige Ergreifung und Verformung des Materials über den Umfang der Verpackungshülse zu erzielen, kann jede Raffbacke mit einer V-förmig sich öffnenden Ausnehmung versehen sein, die zusammen mit der Ausnehmung der gegenüberliegenden Raffbacke einen im wesentlichen rautenförmigen Raffquerschnitt für das Material der Verpackungshülse bildet. Hierdurch wird eine über den Umfang der Verpackungshülse gleichmäßige Durchmesserreduzierung erreicht, ohne daß es über den Umfang verteilt zu unregelmäßigen Materialanhäufungen kommen kann. Der von der V-förmigen Ausnehmung eingeschlossene Winkel beträgt vorzugsweise 45° bis 90°.

Nachstehend ist die Erfindung anhand eines auf der Zeichnung schematisch dargestellten Ausführungsbeispiels einer Vorrichtung zur Durchführung des Verfahrens im einzelnen erläutert. Es zeigen:
- Figur 1: in einer Schnittdarstellung die Vorrichtung mit Tamponhalter, darin eingesetztem Tampon, zwei zueinander verfahrbaren Raffbacken sowie einem Bördelstift;
- Figur 2: die Vorrichtung nach Figur 1 in einer Ansicht von rechts, wobei der Tamponhalter weggelassen ist;
- Figur 3: die Gegenstände nach Figur 1 in einem anderen Verfahrensstadium;
- Figur 4: die Gegenstände nach Figur 2 in einem Verfahrensstadium welches dem Verfahrensstadium nach Figur 3 entspricht und
- Figuren 5a - 5g: in stark vereinfachter Darstellung sieben aufeinanderfolgende Verfahrensschritte des Verfahrens zum Verschließen eine zylindrischen Verpackungshülse.

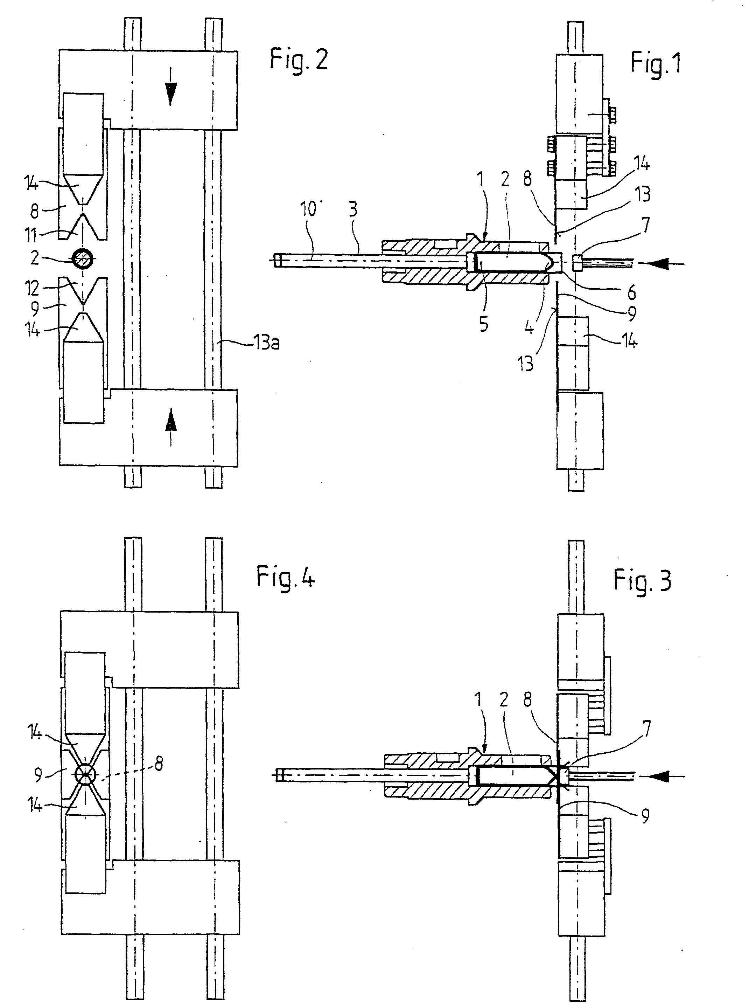

Figur 1 zeigt die Vorrichtung mit einem Tamponhalter 1, einem darin in einer axialen Bohrung angeordnetem Tampon 2 sowie einem axial in dem Tamponhalter 1 geführten Ausstoßer 3. Mittels des als Stößel ausgebildeten Ausstoßers 3 läßt sich der Tampon 2 axial aus dem Tamponhalter 1 herausstoßen, nachdem das im folgenden noch beschriebene Verfahren abgeschlossen ist.

Der Tampon 2 ist in üblicher Weise im wesentlichen zylindrisch geformt mit einem nach Art einer Kuppe oder eines Kegels gestalteten Einführende 4 und einem flachen rückseitigen Ende 5. Das nachfolgend beschriebene Verfahren bezieht sich auf das Verschließen einer den Tampon 2 umgebenden Verpackungshülse im Bereich des Einführendes 4. Die Figuren und insbesondere die Figuren 1 und 3 lassen erkennen, daß der in der Sackbohrung des Tamponhalters 1 angeordnete Tampon 2 von einer Verpackungshülse 6 umgeben ist, die, ebenso wie der Tampon 2, zylindrisch geformt ist. Die Verpackungshülse 6 steht bei dem in Figur 1 dargestellten Verfahrensschritt mit einem zylindrischen Rand über das kuppenförmige Einführende 4 des Tampons 2 hervor. Dieser Rand muß also noch einwärts auf die Kuppe des Tampons 2 angedrückt und versiegelt werden, um die Verpackung des Tampons zu vervollständigen. Zu der Vorrichtung gehört ferner ein Dorn 7, der dem offenen Ende der Verpackungshülse 6 exakt, d. h. koaxial, gegenüberliegt. Der Dorn 7 ist entlang seiner Längsachse axial beweglich und zu diesem Zweck mit einem geeigneten Antrieb versehen, der auf der Zeichnung jedoch nicht dargestellt ist. Der Dorn 7 ist im wesentlichen zylindrisch, sein Durchmesser ist gleich dem Durchmesser des Tampons, kann jedoch auch geringfügig kleiner sein, als der Durchmesser des Tampons.

Zu der Vorrichtung gehören ferner zwei Raffbacken 8, 9, die beim Ausführungsbeispiel oberhalb bzw. unterhalb der Längsachse des Tampons 2 angeordnet sind. Beide Raffbacken 8, 9 sind entlang einer gemeinsamen Bewegungsachse verfahrbar, wobei diese Bewegungsachse die Verlängerung der Längsmittelachse des Tampons 2 rechtwinklig kreuzt. Beide Raffbacken 8, 9 führen daher eine exakt auf die Längsmittelachse des Tampons 2 und damit auch der Verpackungshülse 6 ausgerichtete Bewegung aus. Vorzugsweise erfolgt der Antrieb beider Raffbacken 8, 9 simultan, d. h. mit gleichem Weg und gleicher Geschwindigkeit bis zum Erreichen der Längsmittelachse 10.

Jede Raffbacke 8, 9 besteht aus einer flachen Platte, die, der Längsmittelachse 10 des Tampons zugewandt, mit einer V-förmig sich öffnenden Ausnehmung 11 versehen ist. Die Ausnehmung 11 bildet zusammen mit der Ausnehmung 12 der gegenüberliegenden Raffbacke 9 einen im wesentlichen rautenförmigen Raffquerschnitt für das Material der Verpackungshülse 6. Um dies zu erreichen, ist die größte Weite der identisch gestalteten Ausnehmungen 11, 12 breiter, als der Durchmesser der Verpackungshülse. Außerdem beträgt der von der V-förmigen Ausnehmung 11, 12 eingeschlossene Winkel 45 bis 90°.

Werden die beiden geringfügig zueinander versetzten Raffbacken 8, 9 durch simultane Bewegung von der in Figur 2 dargestellten Ausgangsstellung in die in Figur 4 dargestellt Endstellung gefahren, so gelangen die beiden Raffbacken 8, 9 in zunehmende Überdeckung, wobei die Ausnehmungen 11,12 sich ebenfalls zunehmend überdecken, was zu einer stetigen Verkleinerung des rautenförmigen Querschnittes führt, bis in der Endstellung der beiden Raffbacken 8, 9 nur noch ein geringer freier Querschnitt übrigbleibt. Dieser Endquerschnitt ist gerade so groß, daß das mechanisch zusammengeraffte Material der Verpackungshülse' 6 noch hineinpaßt, ohne daß es infolge der Druckeinwirkung der Raffbacken zu einem Abscheren des Materials kommt. Mit "Raffen" bzw. "Zusammenraffen" ist also gemeint, daß im Bereich der Raffbacken das Material der Verpackungshülse 6 in Richtung auf die Längsmittelachse 10 bewegt wird, und zwar infolge der Mitnahme des Materials durch die auf die Längsmittelachse ausgerichtete Bewegung der Raffbacken 8, 9. Wegen der Verwendung von nur zwei Raffbacken ist die Bewegung des Materials während der Durchmesserreduzierung nicht ausnahmslos radial; vielmehr schiebt sich Material der Verpackungshülse auch an den V-förmigen Kanten der Ausnehmungen 11, 12 der Raffbacken entlang, jedoch wird auch insoweit eine letztlich zu der Längsmittelachse 10 der Verpackungshülse 6 hin führende Bewegung erzielt. Je mehr Raffbacken verwendet werden, desto gleichmäßiger läßt sich das Folienmaterial der Verpackungshülse 6 zur Längsmittelachse 10 hin raffen. Im Idealfall kann mittels einer Vielzahl von Raffbacken eine Art Lamellenblende geschaffen werden, mit der sich das Material über den Umfang betrachtet nahezu gleichmäßig zusammenraffen läßt. Die Kosten einer solchen Konstruktion sind jedoch höher, als bei der in dem Ausführungsbeispiel dargestellten Konstruktion mit nur zwei Raffbacken 8, 9, die zudem auf einer gemeinsamen Führung 13a geführt sind.

Die aus gut wärmeleitendem Material bestehenden Platten, aus denen die Raffbacken 8, 9 zusammengesetzt sind, weisen an den einander zugewandten Seiten Gleitflächen 13 auf, über die sich die Raffbacken 8, 9 unmittelbar gegeneinander abstützen können, so daß zwischen den zusammengefahrenen Raffbacken 8, 9 kein Spalt verbleibt. Erreicht wird hierdurch eine sehr geringe axiale Ausdehnung der Raffbacken 8, 9, deren Platten eine Dicke von 0,7 bis 2 mm aufweisen.

Auf die aus gut wärmeleitendem Material bestehenden Platten sind Heizungen 14 aufgesetzt, mittels denen die Raffbacken 8, 9 zumindest im Bereich der Kanten der V-förmigen Ausnehmungen 11,12 beheizt werden.

Zu der Vorrichtung nach den Figuren 1 bis 4 gehört schließlich noch ein beheizbarer Kopf, der anstelle des Dorns 7 in eine dem Einführende 4 des Tampons 2 axial gegenüberliegende Position gebracht werden kann, um überschüssiges Material der Verpackungshülse in Gestalt einer Rosette thermisch an die bereits geformte Kappe der Verpackungshülse anzubügeln.

Der Ablauf des Verfahrens unter Verwendung der in den Figuren 1 bis 4 dargestellten Vorrichtung wird nachfolgend anhand der Figuren 5a bis 5g erläutert.

Figur 5a zeigt den im Tamponhalter festgehaltenen Tampon 2, während die an beiden Enden noch offene, zylindrische Verpackungshülse 6 über den Tampon gestreift wird. Figur 5b zeigt die schließlich eingenommene Lage der Verpackungshülse 6 auf dem Tampon 2. Diese Lage sollte sich während der anschließenden Verfahrensschritte nicht mehr ändern, wozu Mittel zum vorläufigen axialen Festhalten der Verpackungshülse vorgesehen sind. Diese Mittel sind in Figur 5b durch Druckpfeile F auf Tampon und Verpackungshülse symbolisiert. Spätestens zu diesem Zeitpunkt ist auch der koaxial auf den Tampon aufgerichtete Dorn 7 soweit vorwärtsbewegt worden, daß die im wesentlichen flachen Stirnfläche des Dorns 7 einen Abstand A zu der Kuppe des Tampons aufweist. Der Abstand A sollte geringfügig größer sein, als die summierte Dicke der Raffbacken 8, 9. Die Kante 16 zwischen der zylindrischen Mantelfläche des Dorns 7 und dessen Stirnfläche ist gerundet oder, beim Ausführungsbeispiel, mit einer Anschrägung versehen, um so dem Folienmaterial der Verpackungshülse 6 während des nachfolgenden Raffens weniger Widerstand entgegenzusetzen.

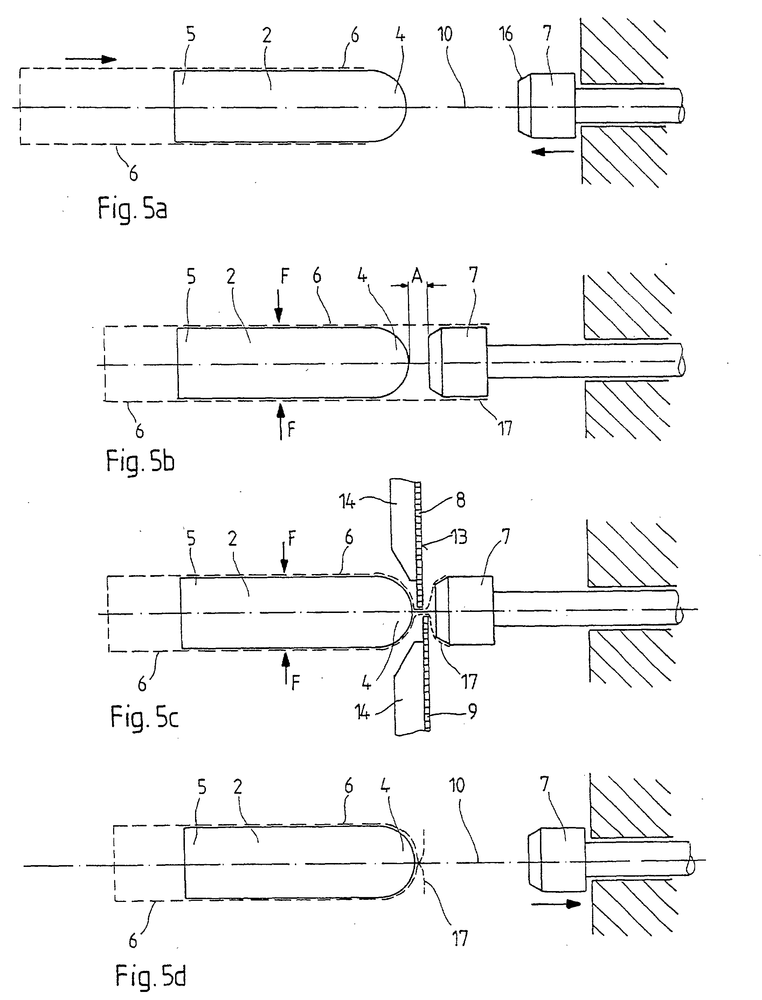

Ist die in Figur 5b dargestellte Stellung des Dorns 7 bezüglich des Tampons 2 bzw. der Verpackungshülse 6 erreicht, werden die Raffbacken 8, 9 zusammengefahren. Figur 5c zeigt die Raffbacken 8, 9 zu jenem Zeitpunkt, zu dem der Endquerschnitt erreicht ist, also das Folienmaterial der Verpackungshülse 6 vollständig zwischen den Raffbacken zusammengerafft ist. Hierbei wird, da eine Relativbewegung zwischen Verpackungshülse 6 und Tampon 2 durch die Kraft F ausgeschlossen ist, Folie, die anfangs noch den Dorn 7 umgibt, zunehmend in den Raffquerschnitt hineingezogen, so daß sich nach Abschluß des in Figur 5c dargestellten Verfahrensschritt keine Folie mehr auf der zylindrischen Mantelfläche des Dorns 7 befindet. Vielmehr bildet das Ende der Folie eine sich im wesentlichen radial vor der Stirnfläche des Dorns 7 erstreckende Rosette 17, wie sie in Figur 5d erkennbar ist.

Infolge der Beheizung der Raffbacken 8, 9 mittels der Heizung 14 kommt es zu einem thermischen Verschmelzen des in dem Raffquerschnitt angesammelten Materials der Verpackungshülse 6. Als besonders geeignet für die Verpackungshülse hat sich beschichtete oder unbeschichtete Folie aus Polypropylen herausgestellt.

Nachdem das in Figur 5c dargestellte Verschweißen der Folie im Raffquerschnitt abgeschlossen ist, werden sowohl die Raffbacken 8, 9, als auch der Dorn 7 zurückgezogen. Dies ist in Figur 5d dargestellt. Zu erkennen ist auch die aufgefächerte Rosette 17 der Verpackungshülse 6, die jetzt um die Kuppe des Tampons 2 herum bereits eine vollständig geschlossene Kappe bildet.

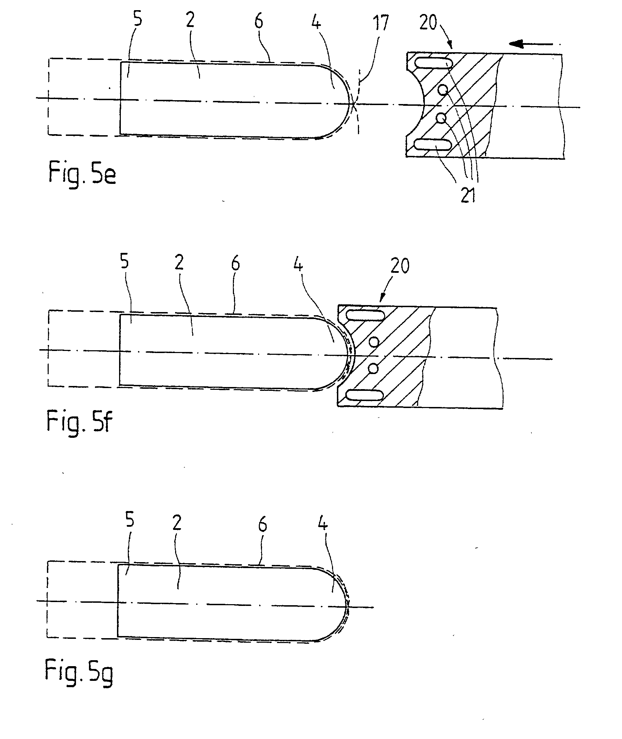

Gemäß Figur 5e wird im nächsten Verfahrensschritt der beheizbare Kopf 20 axial gegen die gebildete Kappe aus Polypropylenfolie gefahren, nachdem zuvor Heizelemente 21 eingeschaltet wurden. In Figur 5f ist die Endstellung des beheizbaren Kopfs 20 dargestellt, in der sich dieser direkt gegen das Ende des Tampons abstützt. Hierzu ist der beheizbare Kopf 20 mit einer konkaven Kontur versehen, die der Kuppenform des Tampons entspricht. Durch die Beheizung des Kopfes 20 tritt eine thermische Versiegelung bzw. ein Anbügeln der Rosette 17 an die bereits gebildete Kappe ein. In Figur 5g ist dargestellt, wie nach dem Zurückziehen des beheizbaren Kopfes 20 der Endabschnitt der Verpackungshülse nahezu flächig auf der Kappe angeschweißt wurde, so daß die Verpackungshülse im Ergebnis nahezu dieselbe Form aufweist, wie das kuppenförmige Ende des Tampons.

In sämtlichen Verfahrensschritten gemäß den Figuren 5a bis 5g werden durch die einzelnen Elemente Dorn 7, Raffbacken 8, 9 und beheizbarer Kopf 20 ausschließlich translatorische Bewegungen ausgeführt. Rotierende Teile sind demgegenüber nicht vorhanden, so daß der gerätetechnische Aufwand der beschriebenen Vorrichtung gering ist. Der Tampon 2 kann auch durch einen Formkörper ersetzt sein, der die Kontur des Tampons nur wiedergibt.

### Bezugszeichenliste

- 1: Tamponhalter
- 2: Tampon
- 3: Ausstoßer
- 4: Einführende des Tampons
- 5: rückseitiges Ende des Tampons
- 6: Verpackungshülse
- 7: Dom
- 8: Raffbacke
- 9: Raffbacke
- 10: Längsmittelachse des Tampons
- 11: Ausnehmung
- 12: Ausnehmung
- 13: Gleitfläche
- 13a: Führung
- 14: Heizung
- 16: Kante
- 17: Rosette
- 20: beheizbarer Kopf
- 21: Heizelement
- A: Abstand
- F: Druckpfeil

## Patentansprüche

1. Verfahren zum Verschließen des einen Endes einer zylindrischen Verpackungshülse (6), insbesondere einer Verpackungshülse zur Aufnahme eines Tampons (2) für die Frauenhygiene, durch Durchmesserreduzierung der Verpackungshülse nahe deren Endes bis zur Bildung einer geschlossenen Kappe, und Ansiegeln des außerhalb der Kappe verbliebenen Endabschnittes der Verpackungshülse an die Außenseite der Kappe,
**dadurch gekennzeichnet,**
**dass** das Material der Verpackungshülse zur Erzielung der Durchmesserreduzierung durch eine zu der Verpackungshülse hin gerichtete Bewegung gerafft und in zusammengerafftem Zustand erhitzt wird, wobei die Erhitzung durch Beheizung über die Raffbacken (8,9) erfolgt.

2. Verfahren nach Anspruch 1, **gekennzeichnet durch** eine radial auf die Längsmittelachse der Verpackungshülse ausgerichtete Bewegung der Raffbacken (8,9).

3. Verfahren nach einem der vorangehenden Ansprüche, **dadurch gekennzeichnet, dass** für die Zeitdauer des Zusammenraffens ein in Bezug auf die Verpackungshülse (6) axial verfahrbarer Dorn (7) in das offene Ende der Verpackungshülse hineinragt.

4. Verfahren nach einem der Ansprüche 1 bis 3, **dadurch gekennzeichnet, dass** sich bei dessen Durchführung der Tampon (2) bereits in der Verpackungshülse (6) befindet.

5. Verfahren nach einem der Ansprüche 1 bis 3, **dadurch gekennzeichnet, dass** sich bei dessen Durchführung in der Verpackungshülse (6) ein Formkörper befindet, der im Bereich der Kappe die Gestalt des zu verpackenden Tampons (2) aufweist.

6. Verfahren nach einem der vorangehenden Ansprüche, **dadurch gekennzeichnet, dass** die außerhalb der Kappe verbliebene Rosette (17) der Verpackungshülse (6) an die Außenseite der Kappe angesiegelt wird, vorzugsweise durch kombinierte Beaufschlagung mit Hitze und Druck.

7. Verfahren nach einem der vorangehenden Ansprüche, **dadurch gekennzeichnet, dass** während des Zusammenraffens nur der die Verpackung bildende Teil der Verpackungshülse (6) in axialer Richtung festgehalten wird, wohingegen dem offenen Endabschnitt der Verpackungshülse axiale Bewegungen möglich sind.

8. Vorrichtung zum Verschließen des einen Endes einer zylindrischen Verpackungshülse (6), insbesondere einer Verpackungshülse zur Aufnahme eines Tampons (2) für die Frauenhygiene, mit Mitteln zum vorläufigen axialen Festhalten der Verpackungshülse sowie Mitteln zur Durchmesserreduzierung der Verpackungshülse (6) nahe deren freien Endes bis zur Bildung einer geschlossenen Kappe,
**dadurch gekennzeichnet,**
**dass** die Mittel zur Durchmesserreduzierung quer zur Längsachse (10) der Verpackungshülse (6) geführte und zu der Verpackungshülse (6) hin bewegliche Raffbacken (8, 9) sind, und dass die Raffbacken (8, 9) beheizbar sind.

9. Vorrichtung nach Anspruch 8, **dadurch gekennzeichnet, dass** die Raffbacken (8, 9) radial zur Längsmittelachse (10) der Verpackungshülse (6) geführt sind.

10. Vorrichtung nach Anspruch 8 oder Anspruch 9, **gekennzeichnet durch** insgesamt zwei Raffbacken (8, 9), welche simultan aufeinander bzw. voneinander weg bewegbar sind.

11. Vorrichtung nach Anspruch 10, **dadurch gekennzeichnet, dass** die Raffbacken (8, 9) mit einander zugewandten Gleitflächen (13) versehen sind, und dass sich die Raffbacken (8, 9) über die Gleitflächen (13) unmittelbar gegeneinander abstützen.

12. Vorrichtung nach Anspruch 10 oder 11, **dadurch gekennzeichnet, dass** jede Raffbacke (8, 9) mit einer V-förmig sich öffnenden Ausnehmung (11 bzw. 12) versehen ist, die zusammen mit der Ausnehmung der gegenüberliegenden Raffbacke einen im wesentlichen rautenförmigen Raffquerschnitt für das Material der Verpackungshülse (6) bildet.

13. Vorrichtung nach Anspruch 12, **dadurch gekennzeichnet, dass** der von der V-förmigen Ausnehmung (11, 12) eingeschlossene Winkel 45° bis 90° beträgt.

## Claims

1. Method of closing one end of a cylindrical packaging sleeve (6), in particular of a packaging sleeve for accommodating a tampon (2) for feminine hygiene, by reducing the diameter of the packaging sleeve in the vicinity of its end until a closed cap is formed, and sealing to the outside of the cap the end portion of the packaging sleeve which remains outside the cap, **characterized in that**, in order to achieve the reduction in diameter, the material of the packaging sleeve is gathered by a movement oriented in the direction of the packaging sleeve and is heated in the gathered-together state, heating taking place via the gathering jaws (8, 9).

2. Method according to Claim 1, **characterized by** a movement of the gathering jaws (8, 9) which is oriented radially in relation to the longitudinal centre axis of the packaging sleeve.

3. Method according to one of the preceding claims, **characterized in that**, for the duration of the gathering-together operation, a mandrel (7), which can be displaced axially in relation to the packaging sleeve (6), projects into the open end of the packaging sleeve.

4. Method according to one of Claims 1 to 3,
**characterized in that**, as it is implemented, the tampon (2) is already located in the packaging sleeve (6).

5. Method according to one of Claims 1 to 3, **characterized in that**, as it is implemented, the packaging sleeve (6) contains a shaped body which, in the region of the cap, is in the form of the tampon (2) which is to be packaged.

6. Method according to one of the preceding claims, **characterized in that** the rosette (17) of the packaging sleeve (6) which remains outside the cap is sealed to the outside of the cap, preferably by being subjected to the combined action of heat and pressure.

7. Method according to one of the preceding claims, **characterized in that**, during the gathering-together operation, only the package-forming part of the packaging sleeve (6) is secured in the axial direction, whereas the open end portion of the packaging sleeve can move axially.

8. Apparatus for closing an end of a cylindrical packaging sleeve (6), in particular of a packaging sleeve for accommodating a tampon (2) for feminine hygiene, having means for temporarily securing the packaging sleeve axially and means for reducing the diameter of the packaging sleeve (6) in the vicinity of its free end until a closed cap is formed, **characterized in that** the diameter-reducing means are gathering jaws (8, 9) which are guided transversely to the longitudinal axis (10) of the packaging sleeve (6) and can be moved in the direction of the packaging sleeve (6), and **in that** the gathering jaws (8, 9) can be heated.

9. Apparatus according to Claim 8, **characterized in that** the gathering jaws (8, 9) are guided radially in relation to the longitudinal centre axis (10) of the packaging sleeve (6).

10. Apparatus according to Claim 8 or Claim 9, **characterized by** a total of two gathering jaws (8, 9), which can be moved simultaneously towards one another and away from one another.

11. Apparatus according to Claim 10, **characterized in that** the gathering jaws (8, 9) are provided with mutually facing sliding surfaces (13), and **in that** the gathering jaws (8, 9) are supported directly against one another via the sliding surfaces (13).

12. Apparatus according to Claim 10 or 11, **characterized in that** each gathering jaw (8, 9) is provided with a recess (11 or 12, respectively) which opens in a V-shaped manner and, together with the recess of the opposite gathering jaw, forms an essentially diamond-shaped gathering cross section for the material of the packaging sleeve (6).

13. Apparatus according to Claim 12, **characterized in that** the angle enclosed by the V-shaped recess (11, 12) is from 45° to 90°.

## Revendications

1. Procédé pour fermer une extrémité d'un manchon cylindrique d'emballage (6), spécialement un manchon d'emballage pour recevoir un tampon (2) pour l'hygiène féminine, par réduction du diamètre du manchon d'emballage à proximité de l'extrémité de celui-ci jusqu'à la formation d'un capuchon fermé, et par scellage de la section d'extrémité du manchon d'emballage restée à l'extérieur du capuchon à la face extérieure du capuchon,
**caractérisé en ce**
**que** le matériau du manchon d'emballage, pour obtenir la réduction du diamètre, est drapé par un mouvement en direction du manchon d'emballage et, à l'état drapé, échauffé, l'échauffement étant réalisé par chauffage par l'intermédiaire des mâchoires de drapage (8,9).

2. Procédé selon la revendication 1, **caractérisé par** un mouvement des mâchoires de drapage (8, 9) dirigé radialement sur l'axe central longitudinal du manchon d'emballage.

3. Procédé selon l'une quelconque des revendications précédentes, **caractérisé en ce que** pendant la durée du drapage un mandrin (7) axialement mobile par rapport au manchon d'emballage (6) saillit dans l'extrémité ouverte du manchon d'emballage.

4. Procédé selon l'une quelconque des revendications 1 à 3, **caractérisé en ce que** lors de la mise en oeuvre de celui-ci, le tampon (2) se trouve déjà dans le manchon d'emballage (6).

5. Procédé selon l'une quelconque des revendications 1 à 3, **caractérisé en ce que** lors de la mise en oeuvre de celui-ci, un corps moulé se trouve dans le manchon d'emballage (6) qui, dans la zone du capuchon, présente la forme du tampon (2) à emballer.

6. Procédé selon l'une quelconque des revendications précédentes, **caractérisé en ce que** la rosette (17) du manchon d'emballage (6) restée à l'extérieur du capuchon est scellée à la face extérieure du capuchon, de préférence par application de chaleur et de pression.

7. Procédé selon l'une quelconque des revendications précédentes, **caractérisé en ce que** pendant le drapage seule la partie du manchon d'emballage (6) formant l'emballage est maintenue dans le sens axial, la section d'extrémité ouverte du manchon d'emballage pouvant exécuter des mouvements axiaux.

8. Dispositif pour fermer une extrémité d'un manchon cylindrique d'emballage (6), spécialement un manchon d'emballage pour recevoir un tampon (2) pour l'hygiène féminine, comprenant des moyens pour la tenue axiale provisoire du manchon d'emballage, ainsi que des moyens pour réduire le diamètre du manchon d'emballage (6) à proximité de l'extrémité libre de celui-ci jusqu'à la formation d'un capuchon fermé,
**caractérisé en ce**
**que** les moyens pour réduire le diamètre sont des mâchoires de drapage (8, 9) guidées perpendiculairement à l'axe longitudinal (10) du manchon d'emballage (6) et mobiles en direction du manchon d'emballage (6), et en ce que les mâchoires de drapage (8, 9) peuvent être chauffées.

9. Dispositif selon la revendication 8, **caractérisé en ce que** les mâchoires de drapage (8, 9) sont guidées radialement à l'axe central longitudinal (10) du manchon d'emballage (6).

10. Dispositif selon la revendication 8 ou la revendication 9, **caractérisé par** au total deux mâchoires de drapage (8, 9) pouvant être déplacées simultanément pour s'approcher ou s'éloigner l'une de l'autre.

11. Dispositif selon la revendication 10, **caractérisé en ce que** les mâchoires de drapage (8, 9) sont munies de surfaces de glissement (13) en regard l'une de l'autre, et **en ce que** les mâchoires de drapage (8, 9) s'appuient directement l'une contre l'autre par les surfaces de glissement (13).

12. Dispositif selon la revendication 10 ou 11, **caractérisé en ce que** chaque mâchoire de drapage (8, 9) est pourvue d'un évidement (11 ou 12) s'ouvrant en forme de V, qui forme, conjointement avec l'évidement de la mâchoire de drapage située en regard, une section transversale de drapage pour l'essentiel en forme de losange pour le matériau du manchon d'emballage (6).

13. Dispositif selon la revendication 12, **caractérisé en ce que** l'angle enfermé par l'évidement (11, 12) en forme de V est compris entre 45° et 90°.
